(19) Europäisches Patentamt
European Patent Office
Office européen des brevets

(11) **EP 3 178 553 B1**

(12) **EUROPEAN PATENT SPECIFICATION**

(45) Date of publication and mention
of the grant of the patent:
**07.08.2019   Bulletin 2019/32**

(21) Application number: **15199100.7**

(22) Date of filing: **10.12.2015**

(51) Int Cl.:
*B01J 23/652* [(2006.01)]     *B01J 35/10* [(2006.01)]
*B01J 37/02* [(2006.01)]     *C07C 29/60* [(2006.01)]
*C07C 31/20* [(2006.01)]

(54) **PROCESS FOR THE MANUFACTURE OF PROPANEDIOL**

VERFAHREN ZUR HERSTELLUNG VON PROPANEDIOL

PROCESSUS DE FABRICATION DE PROPANEDIOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**14.06.2017   Bulletin 2017/24**

(73) Proprietor: **Technip France SA
92400 Courbevoie (FR)**

(72) Inventors:
• **GILLIN, Frédéric
5020 Malonne (BE)**
• **SU, Fangzheng
201100 Minhang district Shanghai (CN)**

• **GILBEAU, Patrick
7090 Braine-le-Comte (BE)**

(74) Representative: **Lavoix
2, place d'Estienne d'Orves
75441 Paris Cedex 09 (FR)**

(56) References cited:
• **FENG JIAN ET AL: "Effect of Base Additives on
the Selective Hydrogenolysis of Glycerol over
Ru/TiO2 Catalyst", CHEMISTRY LETTERS,
CHEMICAL SOCIETY OF JAPAN, JAPAN, vol. 36,
no. 10, 22 September 2007 (2007-09-22), pages
1274-1275, XP008089651, ISSN: 0366-7022, DOI:
10.1246/CL.2007.1274**

**Description**

The invention relates to a process for the manufacture of 1,3-propanediol, in particular for the manufacture of 1,3-propanediol by hydrogenation of glycerol.

Trimethylene glycol (1,3-propanediol) is mainly used as a building block in the production of polymers such as polytrimethylene terephthalate and it can be formulated into a variety of industrial products including composites, adhesives, laminates, coatings, moldings, aliphatic polyesters and copolyesters. It can also be used as a solvent, an ingredient for a food composition, antifreeze and a wood paint. It can also be used in applications such as cosmetics, personal care, cleaning, agricultural compositions, engine coolants, food and beverages, deicing fluids and heat transfer fluids.

Trimethylene glycol may be chemically synthesized by the hydration of acrolein, by the hydroformylation of ethylene oxide to afford 3-hydroxypropionaldehyde, which is hydrogenated to give 1,3-propanediol, by bioprocessing of glucose and glycerol by certain micro-organisms, or by catalytic hydrogenation of glycerol.

For instance, the article "Effect of Base Additives on the Selective Hydrogenolysis of Glycerol over Ru/TiO2 Catalyst" by Feng Jian et al, Chemistry Let, Chemical society of Japan, JAPAN, Vol:36, Nr:10, Page(s):1274 - 1275 describes that glycerol is hydrogenolyzed to 1,2-propanediol using $Ru/TiO_2$ as catalyst in basic aqueous solution under mild reaction conditions (170°C, 3 MPa).

Glycerol hydrogenation has the advantage to use a renewable raw material. However mixtures of propanediols and propanols are obtained which complicates the recovery of 1,3-propanediol especially for industrial scale production. Producing 1,3-propanediol at both high conversion and selectivity remains therefore a challenge. Moreover when a catalyst is used for the hydrogenation of glycerol, catalyst deactivation and subsequent recovery and reactivation remains a problem.

Accordingly, it is desired to provide improved processes for preparing 1,3-propanediol by hydrogenation of glycerol, in particular by catalytic hydrogenation of glycerol.

In a first embodiment, the invention is related to a process for manufacturing 1,3-propanediol from glycerol by reacting glycerol with hydrogen in a reactor in the presence of a supported catalyst, said supported catalyst comprising at least one first element selected from iridium, rhodium, palladium, platinum and any mixture thereof, at least one second element selected from chromium, molybdenum, tungsten, and any mixture thereof, and optionally rhenium, said elements and rhenium being supported on alumina, said process comprising introducing at least one ionic base into said reactor.

One of the essential features of the present invention is the control of the pH of the reaction medium. This allows obtaining at least one of the following advantages:

• An increase of the 1,3 propanediol selectivity or of the 1,3-propanediol yield or of both;
• A decrease of the production of by-products which are difficult to separate from 1,3-propanediol;
• A decrease of the corrosion of the vessel in which the reaction is carried out; and
• A better stability of the catalyst;
• A regulation of catalyst activity by neutralization of acidic impurities in glycerol

Without willing to be bound by any theory, it is believed that the addition of the base to the reactor allows to control the pH of the reaction, which in turn affect the above mentioned process characteristics, in particular when the reaction is carried out in the presence of a supported catalyst comprising at least one first element selected from iridium, rhodium, palladium, platinum and any mixture thereof, at least one second element selected from chromium, molybdenum, tungsten and any mixture thereof, and optionally, rhenium, the elements and rhenium being supported on alumina and most particularly in the presence of a supported catalyst comprising platinum and tungsten, supported on alumina. In that most particularly case, it has been surprisingly observed that the leaching of tungsten from the supported catalyst is reduced.

In a second embodiment, the invention relates to a process for making a polyester comprising obtaining 1,3-propanediol according to the process of the first embodiment and further submitting said 1,3-propanediol to a reaction with a carboxylic acid and/or a carboxylic acid ester.

In a third embodiment, the invention relates to a process for making a polyester fiber comprising obtaining a polyester according to the process of the second embodiment and further converting said polyester in to a fiber.

The present invention therefore relates to processes as defined in the claims. Also disclosed are products obtainable by such processes. Processes and products are illustrated by means of the items listed below:

Item 1. A process for manufacturing 1,3-propanediol from glycerol by reacting glycerol with hydrogen in a reactor in the presence of a supported catalyst, said supported catalyst comprising at least one first element selected from iridium, rhodium, palladium, platinum and any mixture thereof, at least one second element selected from chromium, molybdenum, tungsten, and any mixture thereof, and optionally rhenium, said elements and rhenium being supported on alumina, said process comprising introducing at least one ionic base into said reactor.

Item 2. The process according to item 1, wherein the molar ratio between the ionic base and the glycerol introduced in the reactor is higher than or equal to 0.00005 and lower than or equal to 0.004.

Item 3. The process according to item 2, wherein the molar ratio between the ionic base and the glycerol introduced in the reactor is higher than or equal to 0.00015 and lower than or equal to 0.002

Item 4. The process according to any one of items 1 to 32, wherein the ionic base is added as an alkali metal oxide, an alkaline-earth metal oxide, an alkali metal hydroxide, an alkaline-earth metal hydroxide, ammonium hydroxide, an alkali metal salt of a carboxylic acid, an alkaline-earth metal salt of a carboxylic acid, an ammonium salt of a carboxylic acid or any mixture thereof.

Item 5. The process according to item 4, wherein the alkali metal is sodium.

Item 6. The process according to item 4 or 5, wherein the carboxylic acid is acetic acid.

Item 7. The process according to item 4, wherein the ionic base is added as sodium hydroxide, sodium acetate, or any mixture thereof.

Item 8. The process according to item 7, wherein the ionic base is added as sodium hydroxide.

Item 9. The process according to item 7, wherein the ionic base is added as sodium acetate.

Item 10. The process according to item 9, wherein sodium acetate is added as an aqueous sodium acetate/acetic acid pH buffer.

Item 11. The process according to any one of items 1 to 10, wherein the ionic base is added to the reactor as a mixture with glycerol, water and the catalyst.

Item 12. The process according to item 11, wherein the pH of the mixture is higher than or equal to 3 and lower than 8.

Item 13. The process according to item 12, wherein the pH of the mixture is higher than or equal to 3.5 and lower than or equal to 7.5.

Item 14. The process according to any one of items 1 to 13, wherein the ionic base is added to the reactor as an aqueous solution.

Item 15. The process according to any one of items 1 to 14, comprising withdrawing from the reactor a liquid mixture with a pH higher than or equal to 2.6 and lower than or equal to 7.

Item 16. The process according to item 15, wherein the pH of the withdrawn liquid mixture is higher than or equal to 3.0 and lower than or equal to 6.8.

Item 17. The process according to any one of items 1 to 16, wherein the selectivity of 1,3-propanediol is higher than or equal to 5 mol %.

Item 18. The process according to item 17, wherein the selectivity of 1,3-propanediol is higher than or equal to 25 mol %.

Item 19. The process according to item 18, wherein the selectivity of 1,3-propanediol is higher than or equal to 50 mol %.

Item 20. The process according to any one of items 1 to 19, wherein the selectivity of 1,2-propanediol with respect to glycerol is lower than or equal to 35 mol %.

Item 21. The process according to item 20, wherein the selectivity of 1,2-propanediol is lower than or equal to 30 mol %.

Item 22. The process according to item 21, wherein the selectivity of 1,2-propanediol is lower than or equal to 20 mol %.

Item 23. The process according to any one of items 17 to 22, wherein the first element of the catalyst is platinum and the second element of the catalyst is tungsten.

Item 24. The process according to any one of items 1 to 23, wherein the reactor contains at least one liquid phase, and wherein the reaction is carried out in at least one of the following conditions:

- Said liquid phase contains water in a content higher than or equal to 3 g of water per kg of liquid phase and lower than or equal to 900 g/kg of liquid phase,
- A temperature higher than or equal to 70 °C and lower than or equal to 300 °C; and
- A hydrogen partial pressure higher than or equal to 1 bar absolute and lower than or equal to 200 bar absolute.

Item 25. The process according to item 24 carried out in a slurry reactor.

Item 26. The process according to item 24 carried out in a trickle-bed reactor.

Item 27. The process according to item 24, said process being carried out in the continuous mode, wherein the reaction is carried out in a trickle-bed reactor at a residence time of the liquid phase higher than or equal to 5 min and lower than or equal to 25 h.

Item 28. A process for making a polyester comprising obtaining 1,3-propanediol according to the process of any one of items 1 to 27, and further submitting said 1,3 propanediol to a reaction with a carboxylic acid and/or a carboxylic acid ester.

Item 29. A process for making a polyester fiber comprising obtaining a polyester according to the process of item 28, and further converting said polyester in to a fiber.

Item 30. 1,3-propanediol obtainable by the process of any one of items 1 to 27.

Item 31. Use of the 1,3-propanediol according to item 30 in the manufacture of a polyester.
Item 32. A polyester obtainable by the process of item 31.
Item 33. Use of the polyester according to item 32 in the manufacture of a polyester fiber.

In the process according to the first embodiment of the invention, the reaction may be carried out in a liquid medium or a gaseous reaction medium, preferably in a liquid medium.

The liquid medium may be a single-phase or multi-phase medium.

The liquid medium is composed of all of the dissolved or dispersed solid compounds, dissolved or dispersed liquid compounds and dissolved or dispersed gaseous compounds at the temperature of the reaction.

The liquid medium comprises the reactants, the catalyst, the solvent if any, the impurities present in the reactants, in the solvent if any and in the catalyst, the reaction intermediates, the product and the by-products of the reaction.

The reactants are the glycerol and the hydrogen. The product is 1,3-propanediol. The catalyst, the reaction intermediates, the by-products and the solvent are as described here below.

In the process according to the first embodiment of the invention, when the reaction is carried out in a liquid medium, said liquid reaction medium preferably contains less than 900 g of water per kg of liquid medium. The content of water in the liquid medium is more preferably lower than or equal to 800 g/kg, yet more preferably lower than or equal to 600 g/kg, still more preferably lower than or equal to 400 g/kg, even more preferably lower than or equal to 200 g/kg and most preferably lower than or equal to 100 g/kg. That content of water is usually higher than or equal to 3 g/kg of liquid medium, preferably higher than or equal to 5 g/kg, more preferably higher than or equal to 8 g/kg, yet more preferably higher than or equal to 10 g/kg, still more preferably higher than or equal to 50 g/kg, most preferably higher than or equal to 75 g/kg, yet most preferably higher than or equal to 90g/kg and still most preferably higher than or equal to 95 g/kg. A water content higher than or equal to 200 g/kg and lower than or equal to 800 g/kg, preferably higher than or equal to 250 g/kg and lower than or equal to 600 g/kg, and more preferably higher than or equal to 300 g/kg and lower than or equal to 500 g/kg is also convenient.

Water can be water generated by the hydrogenation reaction or external water e.g. water present in the reactants, the catalyst, the solvent if any or added water.

The water content of the liquid medium can be measured by any method like for instance Karl Fischer titration.

The water content can also be calculated as the sum of the water fed to the liquid medium together with the reactants, e.g. with the glycerol, the catalyst and/or the solvent and the water generated by the hydrogenation reaction. The water generated by the hydrogenation reaction can be obtained from the conversion of glycerol and the selectivity of the various products formed. For instance, each mole of glycerol converted into propanediol will generate one mole of water, each mole of glycerol converted into propanol will generate two moles of water, *etc.*

In the process according to the first embodiment of the invention, the glycerol can be synthetic glycerol or natural glycerol or any mixture thereof. Synthetic glycerol is glycerol which has been obtained from non-renewable raw materials. The glycerol is preferably natural glycerol *i.e.* glycerol which has been prepared in a conversion process of renewable raw materials. By glycerol which has been prepared in a conversion process of renewable raw materials one intends to denote glycerol obtained in a process selected from the group consisting of hydrolysis, saponification, transesterification, aminolysis and hydrogenation of oils and/or fats of animal and/or plant and/or algae origin, fermentation, hydrogenation and hydrogenolysis of mono- and polysaccharides and derived alcohols, derived from or occurring naturally in the biomass, and any combination thereof. Glycerol which has been obtained during the manufacture of biodiesel, *i.e.* during the transesterification of oils and/or fats of animal and/or plant, and preferably during the transesterification of oils and/or fats of plant origin, is particularly convenient. Glycerol which has been obtained in the manufacture of biodiesel is more particularly convenient.

In the process according to the first embodiment of the invention, the hydrogen can be obtained from any source. The hydrogen is preferably molecular hydrogen.

In the process according to the first embodiment of the invention, the hydrogen is preferably obtained from at least one process selected from the group consisting of steam reforming of hydrocarbons, partial oxidation of hydrocarbons, autothermal reforming of hydrocarbons, water-gas shift, coal gasification, pyrolysis of organic waste products (tar, lignite pitch, petroleum distillation residues, plastics, rubber, cellulose, paper, textile, wood, straw, mixed municipal waste...) and co-pyrolysis of organic wastes products with coal (including bituminous coal, lignite,...), thermal and non-thermal plasma cracking of mixtures of water or steam, and fuels, biomass gasification, biomass pyrolysis and subsequent gasification, thermal or catalytic decomposition of nitrogen compounds like ammonia, hydrazine, biochemical hydrogen fermentation, enzymatic treatment of natural sugars (e.g. xylose), steam reforming of alcohols, for instance monoalcohols, such methanol or ethanol, and polyols, such as propanediols and glycerine, alkaline cracking of insaturated fatty acid particularly oleic and ricinoleic acid, electrolysis of an aqueous solution of a hydrogen halide, electrolysis of an aqueous solution of a metal halide like for instance sodium chloride or potassium chloride, hydrolysis of metals or metal hydrides, water splitting from for instance alkaline electrolysis, proton-exchange membrane electrolysis, solid oxide electrolysis, high pressure electrolysis, high temperature electrolysis, photoelectrochemical water splitting, photocatalytic water splitting, photobiological water splitting, solar thermochemical hydrogen production and water thermolysis. When the selected process is electrolysis of an aqueous solution of a hydrogen halide, the hydrogen halide is often selected from hydrogen chloride, hydrogen fluoride and any mixture thereof, and is frequently hydrogen chloride. When the selected process is electrolysis of an aqueous solution of sodium chloride or potassium chloride, electrolysis can be any of mercury electrolysis, membrane electrolysis or diaphragm electrolysis. Membrane electrolysis is preferred.

In the process according to the first embodiment of the invention, the hydrogen can be used in admixture with another compound. The other compound is usually selected from the group consisting of nitrogen, helium, argon, carbon dioxide, steam, saturated hydrocarbons, and any mixture thereof. In the process according to the first embodiment of the invention, the mixture containing hydrogen comprises generally at least 10 % by volume of hydrogen, preferably at least 50 % by volume, more preferably at least 75 % by volume, yet more preferably at least 90 % by volume, still more preferably at least 95 % by volume, most preferably at least 99 % by volume and still most preferably at least 99.9 % by volume. That mixture comprises generally at most 99.99 % of hydrogen by volume. A mixture which consists essentially of hydrogen is also convenient. A mixture which consists of hydrogen is also suitable.

In the process according to the first embodiment of the invention, by alumina, one intends to denote an aluminum hydroxide, an aluminum oxide, an aluminum oxy-hydroxide, any compound resulting from a thermal treatment, especially from a hydrothermal treatment, of aluminum hydroxide, and any mixture thereof, as defined in Ullmann's Encyclopedia of Industrial Chemistry, http://onlinelibrary.wiley.com/doi/10.1002/14356007.a01_557/pdf L.K. Hudson et al., published on line 15 JUN 2000, pages 607-645. Aluminum trihydroxides, aluminum oxide hydroxides and pseudoboehmite are examples of an aluminum hydroxide. Gibbsite, Bayerite or Nordstrandite are examples of an aluminum trihydroxide. Boehmite or Diaspore are examples of an aluminum oxide hydroxide. Corundum is an example of an aluminum oxide. Chi-, kappa-, gamma-, delta-, theta-, and eta-alumina are examples of compound resulting from a thermal treatment, especially a hydrothermal treatment, of an aluminum hydroxide.

In the process according to the first embodiment of the invention, the alumina in the supported catalyst is preferably selected from chi-, kappa-, gamma-, delta-, theta-, eta-alumina, and any mixture thereof, more preferably from gamma-, delta-, theta-alumina, and any mixture thereof, yet more preferably from gamma alumina, delta and theta alumina, still more preferably from gamma alumina and most preferably from delta-, theta-alumina and any mixture thereof. An alumina in the supported catalyst containing less than 10 percent by weight of gamma-alumina is also convenient.

In the process according to the first embodiment of the invention, the alumina can be crystalline, amorphous or a mixture thereof, according to X-ray diffraction analysis. The alumina is preferably a mixture of crystalline and amorphous alumina. X-ray diffraction patterns of crystalline alumina can be found in http://www.sasoltechdata.com/tds/PURALOX_CATALOX.pdf.

In the process according to the first embodiment of the invention, the supported catalyst, preferably exhibits at least one of the following features:

- a nitrogen BET (Brunauer Emmet Teller) specific area higher than or equal to 50 $m^2$/g and lower than or equal to 400 $m^2$/g;
- a nitrogen BET total pore volume higher than or equal to 0.2 $cm^3$/g and lower than or equal to 1.5 $cm^3$/g;
- a nitrogen BET average pore size higher than or equal to 2 nm and lower than or equal to 100 nm;
- a TEM (Transmission Electron Spectroscopy) average particle size of the first compound lower than or equal to 15 nm;
- a content of the at least one first compound with respect to the catalyst higher than or equal to 0.1 percent by weight and lower than or equal to 20 % by weight; and
- a content of the at least one second compound expressed in weight of trioxide per weight of catalyst higher than or equal to 1 percent by weight and lower than 20 percent by weight.

In the process according to the first embodiment of the invention, the first element is preferably selected from palladium, platinum and combination thereof, and is most preferably platinum.

In the process according to the first embodiment of the invention, the content of the first element in the supported catalyst with respect to the catalyst is usually higher than or equal to 0.1 % by weight, preferably higher than or equal to 0.5 % by weight, more preferably higher than or equal to 1 % by weight, yet more preferably higher than or equal to 2 % by weight, and most preferably higher than or equal to 4% by weight. That content is usually lower than or equal to 20 % by weight, preferably lower than or equal to 15% by weight, more preferably lower than or equal to 10% by weight, yet more preferably lower than or equal to 8% by weight and most preferably lower than or equal to 6% by weight. When more than one of the first elements are present, the above mentioned content applies to the sum of the first elements. The weight percent are expressed in weight of metal *i.e.* iridium (Ir) and/or rhodium (Rh) and/or palladium (Pd) and/or platinum (Pt) per weight of catalyst. A catalyst with 2.5 % by weight of Rh and 2.5 % by weight of Pt is an example of a catalyst having a weight percent content of the first element of 5 %. A catalyst with 5 % by weight of Pt is another example of a catalyst having a weight percent content of the first element of 5 %. These features are especially suited when the first element is platinum. A catalyst with a content of about 5 weight percent of the first element, preferably platinum, is particularly convenient.

In the process according to the first embodiment of the invention, the second element is more preferably selected from molybdenum, tungsten, and combination thereof, and is most preferably tungsten.

In the process according to the first embodiment of the invention, the content of the second element in the supported catalyst with respect to the catalyst is usually higher than or equal to 1 % by weight, preferably higher than or equal to 2 % by weight, more preferably higher than or equal to 5 % by weight, yet more preferably higher than or equal to 8 % by weight, and most preferably higher than or equal to 9% by weight. That content is usually lower than 20% by weight, preferably lower than or equal to 17 % by weight, yet more preferably lower than or equal to 15% by weight and most preferably lower than or equal to 11% by weight. When more than one second elements are present, the above mentioned content applies to the sum of the second elements. The weight percent are expressed in weight of metal trioxide, $i.e.$ chromium trioxide ($CrO_3$) and/or, molybdenum trioxide ($MoO_3$) and/or tungsten trioxide ($WO_3$) per weight of catalyst. A catalyst with 5 % by weight of Cr expressed as $CrO_3$ and 5 % by weight of W expressed $WO_3$ is an example of a catalyst having a weight percent content of the second element expressed as trioxide of 10 %. A catalyst with 10 % by weight of W expressed as $WO_3$ is another example of a catalyst having a weight percent content of the second element expressed as metal trioxide compound of 10 %. These features are especially suited when the second element is tungsten. A catalyst with a content of about 10 weight percent of the second element, preferably tungsten, is particularly convenient.

In the process according to the first embodiment of the invention, when rhenium is present in the catalyst, the content of rhenium in the supported catalyst with respect to the catalyst is usually higher than or equal to 0.5 % by weight, preferably higher than or equal to 0.8 % by weight, more preferably higher than or equal to 1 % by weight, yet more preferably higher than or equal to 2 % by weight, and most preferably higher than or equal to 4% by weight. That content is usually lower than 15% by weight, preferably lower than or equal to 10 % by weight, yet more preferably lower than or equal to 8% by weight and most preferably lower than or equal to 6% by weight. The weight percent is expressed in weight of metal $i.e.$ rhenium (Re) per weight of catalyst. A catalyst with a content of about 4 weight percent of rhenium is particularly convenient.

In the process according to the first embodiment of the invention, at least one part of the first element in the supported catalyst is preferably present under the metallic form. The part present under metallic form with respect to the total amount of the first element expressed in weight per cent is preferably higher than or equal to 5 %, more preferably higher than or equal to 10 %, still more preferably higher than or equal to 50 %, yet preferably higher than or equal to 90%, most preferably higher than or equal to 95 %, and yet most preferably higher than or equal to 99 %. The weight percent are expressed as above for the first element. A catalyst where the first element is essentially under metallic form is particularly convenient. These features are especially suited when the first element is platinum.

In the process according to the first embodiment of the invention, at least one part of the second element in the supported catalyst is preferably present as an oxide. The part present under oxide form with respect to the total amount of the second element expressed in weight percent is preferably higher than or equal to 5 %, more preferably higher than or equal to 10 %, still more preferably higher than or equal to 50 %, yet preferably higher than or equal to 90 %, most preferably higher than or equal to 95 %, and yet most preferably higher than or equal to 99 %. The weight percent are expressed as above for the second element. A catalyst where the second element is essentially under oxide form is particularly convenient. These features are especially suited when the second element is tungsten.

In the process according to the invention, at least one part of rhenium in the supported catalyst is preferably present as a low valence rhenium oxide (ReOx). By low valence oxide, it is meant an oxide wherein the rhenium is at an oxidation state of less than or equal to six but not zero. The part of rhenium, present under low valence oxide form with respect the total amount of rhenium compound, expressed in weight percent is preferably higher than or equal to 5 %, more preferably higher than or equal to 10 %, still more preferably higher than or equal to 50 %, yet preferably higher than or equal to 75 %, most preferably higher than or equal to 80 %, and yet most preferably higher than or equal to 90 %. The weight percent are expressed as above for rhenium. A catalyst where rhenium is essentially under low valence oxide form is particularly convenient.

Said contents of the first element, preferably platinum, under metallic form, of the second element, preferably tungsten, under trioxide form and of rhenium, preferably under low valence oxide form, can be determined by X-ray diffraction analysis, Temperature Programmed Reduction and EXFAS as disclosed in Nagawa et al. Journal of Catalysis: 272, 2010, 191-194 and by X-ray Photoelectron Spectroscopy.

In the process according to the first embodiment of the invention, the first element is preferably platinum, more preferably platinum metal, the second element is preferably tungsten, more preferably tungsten trioxide and rhenium is preferably a low valence oxide of rhenium.

In the process according to the first embodiment of the invention, the supported catalyst preferably contains less than 10 weight percent of titanium expressed as titanium dioxide.

In the process according to the first embodiment of the invention, the catalyst is usually in a non-powder form selected from the group consisting of rings, beads, spheres, saddles, pellets, tablets, extrudates, granules, crushed, flakes, honeycombs, filaments, cylinders, polygons and any mixture thereof, or in powder form. The preferred form depends usually of the type of reactor used. A powder form is preferred when a slurry or fluidized-bed reactor is used for carrying out the reaction, while non-powder forms are preferred when a fixed bed reactor or a trickle bed reactor is used for carrying out the reaction.

In the process according to the first embodiment of the invention, the reaction may be carried out in the absence or in the presence of a solvent. The solvent may be selected from the group consisting of inorganic solvent, organic solvent, and combinations thereof. Examples of inorganic solvents are water, supercritical carbon dioxide, and inorganic ionic liquids. Examples of organic solvents are alcohols, ethers, saturated hydrocarbons, esters, perfluorinated hydrocarbons, nitriles, amides and any mixture thereof. Examples of alcohols are methanol, ethanol, 1-propanol, 2-propanol, ethylene glycol, 1,2-propanediol, 1,3-propanediol. Examples of ethers are diethylene glycol, dioxane, tetrahydrofuran, ethylene glycol monomethyl ether, ethylene glycol monoethyl ether, ethylene glycol dimethyl ether, diethylene glycol monomethyl ether and diethylene glycol dimethyl ether. An example of saturated hydrocarbons is cyclohexane. An example of esters is ethyl acetate. Examples of perfluorinated hydrocarbons are perfluorinated alkane such as perfluorinated hexane, heptane, octane, nonane, cyclohexane, methylcyclohexane, dimethylcyclohexane or trimethylcyclohexane, perfluoroethers like Galden® HT from Solvay Solexis, perfluorotetrahydrofurane or perfluoroamine like FluorinertTM FC from 3M. An example of nitriles is acetonitrile. An example of amides is dimethylformamide.

In the process according to the first embodiment of the invention, when the reaction is carried out in the presence of a solvent, different from water, the content of the solvent in the liquid medium is usually higher than or equal to 1 g/kg of liquid reaction medium, preferably higher than or equal to 2 g/kg, more preferably higher than or equal to 5 g/kg, yet more preferably higher than or equal to 10 g/kg, still more preferably higher than or equal to 50 g/kg, most preferably higher than or equal to 100 g/kg, yet most preferably higher than or equal to 150g/kg and still most preferably higher than or equal to 200g/kg. That content of solvent is usually lower than or equal to 999 g/kg, preferably lower than or equal to 950 g/kg, more preferably lower than or equal to 900 g/kg, yet more preferably lower than or equal to 850 g/kg, still more preferably lower than or equal to 825g/kg and most preferably lower than or equal to 800 g/kg.

In the process according to the first embodiment of the invention, the reaction may also be carried out solventless, *i.e.* for a content of a solvent, different from water, from the product of reaction and from the by-products of reaction , in the liquid medium lower than 1 g/kg.

In the process according to the first embodiment of the invention, at least one ionic base is added to the reactor where the reaction between glycerol and hydrogen occurs.

A base according to the process of the invention is a substance that can combine with a proton (hydrogen ion) to form a new compound.

Non limiting examples of ionic bases are the oxide ion, the hydroxide ion, the carbonate and hydrogen carbonate ions, the phosphate, hydrogen phosphate and dihydrogen phosphate ions and the conjugate bases of carboxylic acids. The ionic base are preferably added as molecular entities containing the ionic bases. The definition of ionic bases covers such molecular entities containing the inorganic bases. Non limiting examples of such molecular entities are alkali and alkaline-earth metal oxides, alkali and alkaline-earth metal hydroxides, ammonium hydroxide, alkali salts of carboxylic acids, alkaline-earth metal salts of carboxylic acids, ammonium salts of carboxylic acids and any mixture thereof.

In the first embodiment according to the invention, the inorganic base, is preferably selected from alkali and alkaline-earth metal oxides, alkali and alkaline-earth metal hydroxides, ammonium hydroxide, alkali salts of carboxylic acids, alkaline-earth metal salts of carboxylic acids, ammonium salts of carboxylic acids and any mixture thereof. Sodium hydroxide and sodium acetate are more preferred bases with sodium hydroxide being the most preferred. In the process according to the invention, the ionic base may be in the form of a liquid, an essentially anhydrous solid, a hydrated solid, an aqueous and/or organic solution or an aqueous and/or organic suspension. The ionic base is preferably in the form of an essentially anhydrous solid, a hydrated solid, an aqueous solution or an aqueous suspension.

The expression "essentially anhydrous solid" is understood to mean a solid of which the water content is less than or equal to 20 g/kg, preferably less than or equal to 10 g/kg and more preferably less than or equal to 1 g/kg.

The expression "hydrated solid" is understood to mean a solid of which the water content is at least 20 g/kg and at most 700 g/kg, preferably at least 50 g/kg and at most 650 g/kg and most particularly preferably at least 130 g/kg and at most 630 g/kg. The hydrates which denote solid combinations of substances with one or more water molecules are examples of hydrated solids.

When the ionic base is used in the form of an aqueous solution, its content in the aqueous solution is generally greater than 20 g/kg, preferably greater than or equal to 70 g/kg and more preferably greater than or equal to 150 g/kg. This content is generally less than or equal to the solubility of the ionic base in water at the reaction temperature of step a).

When the ionic base is used in the form of an aqueous suspension, its content in the aqueous suspension is generally greater than the solubility of the base in water at the reaction temperature of step a), preferably greater than or equal to 20 g/kg and more preferably greater than or equal to 70 g/kg. This content is generally less than or equal to 400 g/kg, preferably less than 300 g/kg.

The preferred ionic bases are in the form of concentrated aqueous solutions or suspensions of sodium hydroxide.

The sodium hydroxide content of solutions or suspensions of sodium hydroxide is generally greater than or equal to 30 g/kg, usually greater than or equal to 40 g/kg, particularly greater than or equal to 60 g/kg, in many cases greater than or equal to 100 g/kg, and preferably greater than or equal to 120 g/kg. This sodium hydroxide content is generally less than or equal to 500 g/kg, commonly less than or equal to 350 g/kg, often less than or equal to 200 g/kg and advantageously less than or equal to 160 g/kg. Contents of 125, 130, 135, 140, 145, 150 and 155 g/kg are particularly convenient.

In the process according to the invention, the molar ratio between the base and the glycerol introduced to the reactor is preferably higher than or equal to 0.00005, more preferably higher than or equal to 0.00010, and most preferably higher than or equal to 0.00015. This ratio is preferably lower than or equal to 0.004, more preferably lower than or equal to 0.003, and most preferably lower than or equal to 0.002.

In the process according to the invention, when the ionic base is sodium acetate, said ionic base is preferably introduced into the reactor as an aqueous sodium acetate/acetic acid pH buffer.

The ionic base can be introduced to the reactor by any way. Preferred way of introduction are independently of the other reactants, in admixture with glycerol, in admixture with the catalyst, in admixture with the solvent, if any, in admixture with water if any, or any combination thereof.

The base can also be introduced in a stream which is recycled to the reactor. This is particularly suitable when the process is carried out in a continuous way.

A preferred way to add the ionic base to the reactor is as a mixture with glycerol, water and the catalyst. In that case, the pH of said added mixture is preferably higher than or equal to 3, more preferably higher than or equal to 4 and most preferably higher than or equal to 4.5. This pH is preferably lower than 8, more preferably lower than or equal to 7.5 and most preferably lower than or equal to 7.0. This way to add the ionic base to the reactor is particularly well suited when the process is carried out batchwise *i.e.* under discontinuous mode.

The process according to the first embodiment of the invention usually comprises withdrawing from the reactor a liquid mixture. In that case, the pH of said withdrawn is preferably higher than or equal to 2.6, more preferably higher than or equal to 3.5 and most preferably higher than or equal to 4. This pH is preferably lower than 7, more preferably lower than or equal to 6 and most preferably lower than or equal to 5.

The pH measurement is preferably carried out by bringing the withdrawn liquid mixture at ambient temperature and a pressure of 1 bar absolute.

When the process is carried out under continuous mode, monitoring the pH of the withdrawn mixture is a way to adjust the amount of ionic base to be added to the reactor.

The pH measurement can be carried out by any means. Measurement with a pH sensitive electrode is convenient. Such an electrode should be stable in the reaction medium under the measurement conditions and should not contaminate the reaction medium. Glass electrodes for measuring pH are more particularly convenient. Examples of such electrodes are given in Ullmann's Encyclopedia of Industrial Chemistry,© 2005, Wiley-VCH Verlag GmbH & Co. KGaA, Weinheim 10.1002/14356007.e19_e01, pp. 8-15. Electrodes of the type 405 DPAS-SC-K85 supplied by METTLER TOLEDO® or of the types Ceragel CPS71 and Orbisint CPS11 supplied by ENDRESS + HAUSER® or HI1230 supplied by HANNA® are examples of electrodes that can be used.

When the process is carried out continuously, the pH of the added mixture and of the withdrawn mixtures can be measured either continuously or periodically. In this last case, the measurement is usually carried out at a frequency sufficiently high to maintain the pH in the set ranges during at least 80 % of the reaction time, often during at least 90 %, frequently during at least 95 % and in particular during at least 99 %.

The process according to the first embodiment of the invention can be carried out according to any mode of operation. The mode of operation can be continuous or discontinuous. By continuous mode of operation, one intends to denote a mode of operation where the glycerol and hydrogen are added continuously in the process, and the 1,3-propanediol is continuously withdrawn from the process. Any other mode of operation is considered as discontinuous.

In the process according to the first embodiment of the invention, the reaction is preferably carried out in a liquid medium.

The process according to the first embodiment of the invention can be carried out in reaction apparatuses made of or coated with materials which are suitable for hydrogenation under pressure, resistant in the presence of corrosive compounds under the reaction conditions. Suitable materials can be selected from the group consisting of glass, enamel, enameled steel, graphite, impregnated graphite, like for example graphite impregnated with a perfluorinated polymer such as polytetrafluoroethylene, or graphite impregnated with a phenolic resin, polyolefins, like for instance polyethylene or polypropylene, fluorinated polymers, like perfluorinated polymers such as for instance polytetrafluoroethylene, poly(perfluoropropylvinylether), copolymers of tetrafluoroethylene and hexafluoropropylene, and like partially fluorinated polymers such as for instance poly(vinylidene fluoride), polymers comprising sulphur, like for instance polysulphones or polysulphides, metals, like for instance tantalum, titanium, copper, gold, silver, nickel and molybdenum, and metal alloys, like for instance serie 300 stainless steel such as stainless steel type 302, stainless steel type 304, stainless steel type 316, alloys containing nickel, such as Hastelloy B, Hastelloy C, alloys containing molybdenum, such as Inconel 600, Inconel 625 or Incoloy 825.

The materials may be used within the mass, or in the form of cladding, or else by means of any coating process. Enameled steel is particularly convenient. Glass-lined apparatuses are also convenient. Stainless steel type 316 and Hastelloy C and tantalum coated apparatuses are also convenient.

In the process according to the first embodiment of the invention, the reaction is carried out at a temperature preferably higher than or equal to 70°C, more preferably higher than or equal to 80°C, yet more preferably higher than or equal to 90°C and most preferably higher than or equal to 100°C. That temperature is preferably lower than or equal to 300°C, more preferably lower than or equal to 200°C, yet more preferably lower than or equal to 180°C and most preferably lower than or equal to 150°C. A temperature between 150 °C and 180°C is also particularly convenient.

In the process according to the first embodiment of the invention, the reaction is preferably carried out at a hydrogen partial pressure preferably higher than or equal to 1 bar absolute (1 bara), more preferably higher than or equal to 5 bara and yet more preferably higher than or equal to 10 bara, still more preferably higher than or equal to 20 bara, most preferably higher than or equal to 50 bara, and yet most preferably higher than or equal to 80 bara. That hydrogen partial pressure is preferably lower than equal to 200 bara, more preferably lower than equal to 150 bara and most preferably lower than equal to 120 bara. A pressure between 50 bara and 70 bara is also particularly convenient.

The process according to the first embodiment of the invention may be carried out in any type of reactor. The reactor may be selected from the group consisting of a slurry reactor, a fixed bed reactor, a trickle bed reactor, a fluidized bed reactor, and combinations thereof. A slurry reactor or a trickle bed reactor is particularly convenient. A trickle bed reactor is more particularly suitable. A trickle bed reactor fed at co-current by hydrogen and the glycerol is very particularly convenient. One or more reactors could be used.

In the process according to the first embodiment of the invention carried out under continuous mode, the residence time which is the ratio of the volume of the liquid medium in the reactor to the flow rate by volume of the liquid reactants, depends on the reaction rate, on the hydrogen partial pressure, on the temperature, on the thoroughness with which the liquid medium is mixed, and on the activity and concentration of the supported catalyst. This residence time is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 20 hours, frequently lower than or equal to 10 and in particular lower than or equal to 5 hours.

In the process according to the first embodiment of the invention carried out under continuous mode, especially when a gas and a liquid phase are present, the residence time for the liquid phase, which is the ratio of the volume of the reactor volume to the flow rate by volume of the liquid phase, is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 10 hours and frequently lower than or equal to 5 hours.

In the process according to the first embodiment of the invention carried out under continuous mode, especially when a gas and a liquid phase are present, the residence time for the gas phase, which is the ratio of the volume of the reactor to the flow rate by volume of the gas phase, is usually higher than or equal to 1 second, often higher than or equal to 3 seconds, frequently higher than or equal to 7 seconds and in particular higher than or equal to 10 seconds. This residence time is usually lower than or equal to 10 minutes, often lower than or equal to 5 minutes and frequently lower than or equal to 2 minutes.

In a Continuous Stirred-Tank Reactor or in a bubble column, the volume of the reactor in the above definition of the residence time of the liquid or the gas phase can be replaced by the volume of the reactor occupied by the liquid.

In a trickle-bed reactor, the volume of the reactor in the above definition of the residence time of the liquid or the gas phase can be replaced by the volume of the reactor occupied by the catalyst.

In the process according to the first embodiment of the invention carried out under discontinuous mode, the reaction time required for the process according to the invention depends on the reaction rate, on the hydrogen partial pressure, on the glycerol concentration, on the temperature, on the thoroughness with which the reaction mixture is mixed, and on the activity and concentration of the supported catalyst. The required reaction time is usually higher than or equal to 5 minutes, often higher than or equal to 15 minutes, frequently higher than or equal to 30 minutes, in particular higher than or equal to 60 minutes, and more specifically higher than or equal to 160 min. This reaction time is usually lower than or equal to 25 hours, often lower than or equal to 20 hours, frequently lower than or equal to 10 and in particular lower than or equal to 6 hours.

In the process according to the first embodiment of the invention carried out under discontinuous mode, especially when a gas and a liquid phase are present, the reaction time for the liquid phase is usually higher than or equal to 5 minutes, often higher than or equal to 15 minutes, frequently higher than or equal to 30 minutes, in particular higher than or equal to 60 minutes, and more specifically higher than or equal to 180 min. This reaction time is usually lower than or equal to 25 hours, often lower than or equal to 10 hours and frequently lower than or equal to 6 hours.

In the process according to the first embodiment of the invention carried out under discontinuous mode, especially when a gas and a liquid phase are present, the reaction time can be adjusted depending on the conversion sought for the glycerol and the selectivity sought for the 1,3-propanediol, under the reaction conditions, and that reaction time can vary between 6 and 80 h.

In the process according to the first embodiment of the invention, especially when the reaction is carried out continuously, the molar ratio between the flow rates of hydrogen and glycerol is usually higher than or equal to 0.1 mol/mol, often higher than or equal to 0.5 mol/mol, frequently higher than or equal to 0.7 mol/mol and in particular higher than 1 mol/mol. This ratio is usually lower than or equal to 100 mol/mol, often lower than or equal to 50 mol/mol, frequently lower than or equal to 20 mol/mol and in particular lower than or equal to 10 mol/mol.

In a simple way of carrying out the process according to the first embodiment of the invention, the process can be carried out discontinuously, in the following manner. An autoclave which is provided with a stirring or mixing unit and which can be thermostated is charged, in a suitable manner, with glycerol to be hydrogenated, the catalyst and a possible solvent. Thereafter, hydrogen is forced in until the desired pressure is reached, and the mixture is heated to the chosen reaction temperature while mixing thoroughly. The course of the reaction can be readily monitored by measuring the amount of hydrogen consumed, which is compensated by feeding in further hydrogen until the targeted conversion for glycerol is reached.

The mixture present after the hydrogenation can be worked up, for example, as follows : when the hydrogenation is complete, the reaction vessel is cooled, the pressure is let down and the catalyst is filtered off and rinsed with the possible solvent used, and the possible solvent used is then removed under reduced pressure or under atmospheric pressure. The crude product which remains can likewise be purified further by distillation under reduced pressure or under atmospheric pressure. When high-boiling solvents are used, it is also possible first to distil off the propanediol. When no solvent is used the mixture can be submitted directly to a distillation under reduced pressure or under atmospheric pressure. The recovered catalyst e.g by filtration can be reused in the discontinuous process as such or after reactivation by a physico-chemical treatment.

In another way of carrying out the process according to the first embodiment of the invention, the process can be carried out continuously, in the following manner: a vertical cylindrical reactor which can be thermostated is charged with the catalyst provided in a suitable shape in order to obtain a fixed bed of catalyst in the reactor. The reactor is fitted on its top part with inlet ports to feed the glycerol to be hydrogenated, neat or dissolved in a solvent and hydrogen, on its bottom part with an outlet port to recover the reaction mixture, with a regulation pressure device, and a vessel, to recover the reaction mixture and separate the liquid phase and the gas phase. Thereafter, hydrogen is introduced in the reactor until the desired pressure is reached, then the reactor is heated to the chosen reaction temperature, hydrogen and the glycerol, neat or dissolved in a solvent, are forced continuously in the reactor at the selected molar ratio and the reaction mixture is collected continuously in the recovery vessel. The extent of the reaction can be readily monitored by analyzing the composition of the liquid separated in the collection vessel. The liquid mixture present in the recovery vessel can be worked up, for example, by distillation under reduced pressure or under atmospheric pressure.

When the process according to the first embodiment of the invention is carried out continuously, a continuous process purge of gaseous by-products of the reaction or of gaseous contaminants of raw materials may be present and the main part of the non reacted hydrogen can be recycled to the reactor.

Gas chromatography is usually used for assessing the content of the organic compounds in samples withdrawn at various stages of the process.

The present invention also relates in a second embodiment to a process for making a polymer selected from the group consisting of a polyether, a polyurethane, a polyester, and any mixture thereof, comprising obtaining 1,3-propanediol from the process according to the first embodiment, and further using said 1,3-propanediol as raw materials.

The process for making the polyether comprises obtaining 1,3-propanediol according to the first embodiment, and further submitting said 1,3-propanediol to a reaction with at least one compound selected from the group consisting of a halogenated organic compound, an organic epoxide, an alcohol, or any mixture thereof.

The process for making the polyurethane comprises obtaining 1,3-propanediol according to the first embodiment, and further submitting said 1,3-propanediol to a reaction with a polyisocyanate, preferably a diisocyanate.

In the process for making the polymer according to the invention, the polymer is preferably a polyester.

The present invention therefore also relates to a process for making a polyester comprising obtaining 1,3-propanediol according to the first embodiment and further submitting said 1,3-propanediol to a reaction with a carboxylic acid and/or a carboxylic acid ester.

The features mentioned above for the process for manufacturing the 1,3-propanediol are applicable for obtaining the 1,3-propanediol used for making the polymer, preferably a polyester.

In the process for making a polyester according to the invention, the carboxylic acid is preferably a polycarboxylic acid, more preferably a dicarboxylic acid. The polycarboxylic acid is preferably selected from the group consisting of an aliphatic acid, saturated or unsaturated, an aromatic acid, an alkylaromatic acid, saturated or unsaturated, a heteroaromatic acid, an alkylheteroaromatic acid, saturated or unsaturated, or any mixture thereof.

The preferred aliphatic dicarboxylic acid contains from 2 to 16 carbon atoms and is more preferably selected from the group consisting of oxalic acid, malonic acid, succinic acid, glutaric acid, adipic acid, sebacic acid, azelaic acid and any mixture thereof.

The preferred unsaturated dicarboxylic acid is preferably selected from the group consisting of fumaric acid, maleic acid, and any mixture thereof.

The preferred aromatic dicarboxylic acid is preferably selected from the group consisting of o-phthalic acid, m-phthalic acid, p-phthalic acid (terephthalic acid), naphthalene dicarboxylic acid, and any mixture thereof. The preferred aromatic dicarboxylic acid is more preferably terephthalic acid.

The preferred alkyl aromatic dicarboxylic acid is preferably selected from the group consisting of 4-methylphthalic acid, 4-methylphthalic acid, and any mixture thereof.

The preferred unsaturated dicarboxylic aromatic acid is preferably selected from the group consisting of vinyl phthalic acids, and any mixture thereof.

The preferred heteroaromatic dicarboxylic acid is preferably selected from the group consisting of furano dicarboxylic acids, and any mixture thereof, and is preferably 2,5-furano dicarboxylic acid.

In the process for making a polyester according to the invention, the carboxylic acid ester is preferably an ester of the above cited dicarboxylic acid, preferably a methyl, or ethyl ester. The preferred ester is selected from the group consisting of an ester of terephthalic acid, an ester of furano dicarboxylic acid, and any mixture thereof. The ester is more preferably a terephthalic acid ester, and most preferably dimethyl terephthalate.

The production of the polyester is described, for example, in Ullmann's Encyclopedia of Industrial Chemistry, Horst Köpnick, Manfred Schmidt, Wilhelm Brügging, Jörn Rüter and Walter Kaminsky, Published Online : 15 JUNE 2000, DOI : 10.1002/14356007.a21_227, pages 623-649.

The polymer, preferably a polyester, obtained according to the process of the invention usually exhibits a $^{14}C/^{12}C$ higher than or equal to $0.33 \ 10^{-12}$, often higher than or equal to $0.5 \ 10^{-12}$, frequently higher than or equal to $0.75 \ 10^{-12}$, in many case higher than or equal to $1.0 \ 10^{-12}$ and in particular higher than or equal to $1.1 \ 10^{-12}$.

In a further embodiment, the invention also relates to a polyester exhibiting a $^{14}C/^{12}C$ higher than or equal to $0.33 \ 10^{-12}$, often higher than or equal to $0.5 \ 10^{-12}$, frequently higher than or equal to $0.75 \ 10^{-12}$, in many case higher than or equal to $1.0 \ 10^{-12}$ and in particular higher than or equal to $1.1 \ 10^{-12}$.

The polyester is preferably obtainable by reacting 1,3-propanediol obtained from the process according to the first embodiment, and further submitting said 1,3-propanediol to a reaction with a carboxylic acid and/or a carboxylic acid ester, as described here above.

The polyester is more preferably obtained by reacting 1,3-propanediol obtained from the process according to the first embodiment, with a carboxylic acid and/or a carboxylic acid ester, as described here above.

The present invention also relates in a third embodiment to a process for making a polyester fiber comprising obtaining 1,3-propanediol from the process according to the first embodiment, submitting said 1,3-propanediol to a reaction with a carboxylic acid and/or a carboxylic acid ester to obtain a polyester according to the second embodiment and further converting said polyester into a fiber.

The methods for measuring the $^{14}C$ content are precisely described in standards ASTM D 6866 (notably D 6866-06, D 6866-08 and D 6866-12) and in standards ASTM 7026 (notably D 7026-04). The method preferably used is described in standard ASTM D6866-12.

The features mentioned above for the process for manufacturing the 1,3-propanediol and for the process for making the polyester are applicable for obtaining the 1,3-propanediol and the polyester used for making the polyester fiber.

The production of the polyester fibers is described, for example, in Ullmann's Encyclopedia of Industrial Chemistry, Helmut Sattler and Michael Schweizer, Published Online : 15 OCT 2011, DOI : 10.1002/14356007.o10_o01, pages 1 to 34.

The polyester fibers have numerous applications and can be used in tires, rope, cordage, sewing thread, seat belts, hoses, webbing, coated fabrics, carpets, apparel, home fashions, upholstery, medical, interlinings, filtration, fiberfill, high-loft, roofing, geotextiles, and substrates, for instance.

In a fourth embodiment, the invention relates to 1,3-propanediol obtainable according to the process of the first embodiment.

In a fifth embodiment, the invention also relates to the use of the 1,3-propanediol of the fourth embodiment in the manufacture of a polyester.

The features mentioned above for the process for manufacturing the polyester according to the second embodiment are applicable to the use of the fifth embodiment.

In a sixth embodiment, the invention also relates to a polyester obtainable according to the process of the fifth embodiment.

The features mentioned above for the polyester obtained according to the second embodiment are applicable to the polyester of the sixth embodiment.

In a seventh embodiment, the invention relates to the use of the polyester of the sixth embodiment in the manufacture of a polyester fiber.

The examples below are intended to illustrate the invention without, however, limiting it.

Examples 1 to 6

Catalyst preparation

The supported catalyst was prepared by the successive wet-impregnating method. The support was impregnated with an aqueous solution (5 g $H_2O$/g support) of ammonium metatungstate hydrate (Aldrich) at about 25 °C for about 60 minutes, under agitation in a rotary evaporator. The water was removed by using rotary evaporator at about 50 °C, at about 23 mbara (vacuum with a water jet pump at 20°C) and for about 30 minutes, and the resulting sample was dried overnight at about 110 °C under static air under 1 bara. The dried sample was then calcined at 450 °C under static air, at 1 bara, for 3 hours. The calcined sample was slurried with an aqueous solution of chloroplatinic acid hydrate (Aldrich, ∼ 38% Pt basis) (5 g $H_2O$/g calcined sample). The water was removed as above, the sample containing platinum was dried at 110 °C as above, calcined at 450 °C as above, and treated at 300 °C with flowing hydrogen (2.2-2.9 mlN/min/g of catalyst) for 3 hours. The following alumina based oxide has been used as: theta-$Al_2O_3$ (Puralox TH 100/90) was provided by Sasol as powder and used as received. The catalysts have been obtained as powders.

The weight ratio of Pt and $WO_3$ in the final Pt/$WO_3$/$Al_2O_3$ catalysts were respectively of 5.1 wt% (Pt) and 9.4 wt% ($WO_3$).

Test conditions

The hydrogenation of glycerol has been carried out in a 300 ml polytetrafluoroethylene lined autoclave.

4.5 g catalyst, 90 g of a mixture of glycerol (VWR, vegetal origin, min. 99,5% containing 2.15 g/kg of water), 60 g of water (Milli-Q water) and various amounts of a NaOH (Sigma Aldrich, 98%) aqueous solution (1-3.1 wt /wt%) have been placed in the autoclave. Subsequently, the autoclave was sealed and purged with nitrogen (N2, Air Product, 99.998%) by pressurizing and depressurizing several times and then with hydrogen ($H_2$, Air Product, 99.9995%) by pressurizing and depressurizing several times, and then heated to the desired temperature of 180 °C under mechanical stirring of about 500 rpm and about 10 bara of hydrogen . The hydrogen pressure was then increased to 60 bara and the mechanical stirring was set at about 1000 rpm. The time zero for the reaction was defined as the time at which the stirring was increased. During the reaction, the pressure was always maintained at 60 bara. After a reaction time of 6 h, the reaction was stopped by cooling down the autoclave. The pressure was released and the gas phase was sampled by flowing in a metal container and then analyzed by gas chromatography (GC). The liquid product was recovered and its pH was measured. The liquid product was then further separated by centrifugation and then analyzed by gas chromatography (GC).

3. Analyses

The pH of the liquid product has been measured at ambient temperature and corrected to 25 °C by using a glass electrode (HANNA HI1230) previously calibrated with two aqueous buffer at the same temperature (Sigma, buffer reference standards B4770 (pH 4.00 +/- 0.01 at 25°C) and B5020 (pH 7.00+/- 0.01 at 25°C).

The GC analyses of the liquids have been carried out under the following conditions. The sample has been dissolved in dimethylformamide. The GC has been has been performed by injecting the solution on a CP Wax 57 CB column (25m*0.25mm*0.2um) using an appropriate temperature program and flame ionisation detection. Quantification has been done using an internal standard (2-chlorotoluene) and relative response factors, determined using standard reference products. For unidentified products (with unknown molecular formula) and for products with known molecular formula but with unknown structure , the relative response factor of 1,3-propanediol has been used. The specific GC conditions were as follows:

- Injector temperature: 250 °C
- Detector temperature: 280 °C
- Oven temperature: 60 °C (5 min) - 10 °C/min - 240 °C (5 min)
- Split flow: 60 ml/min
- Flow rate: 1 ml/min (constant)

For the unidentified products, the carbon number and the molecular mass were by convention respectively 6 and 92.

The GC analyses of the gas has been carried out by injecting the gas sample with a gas sampling valve and a sampling loop on a porous layer open tubular Molesieve 5A capillary column (30m x 0.53mm x 50 um) using an appropriate temperature program and a thermal conductivity detector. Quantification is done by external standard method and the absolute response factors are determined by using fixed gas mix standard supplied by Air Products. The specific GC conditions were as follows:

- Valve temperature: 110 degrees centigrade
- Detector temperature:
- Block Temperature 300 degrees centigrade
- Transfer line: 140 degrees centigrade
- Filament temperature: 260 degrees centigrade
- Filament Voltage: 10 V
- Flow:
- Make up: 15 ml/min
- Reference: 30 ml/min.
- Oven temperature: 70 degrees centigrade (6 min) - 8 degrees C/min - 180 degrees centigrade (20 min)
- Column Pressure: 100 KPa. The mass balance was calculated for each test and it was found higher than 95 % in most cases.

The conditions for carrying out examples 1 to 6 and the results are summarized in Table 1 here below.

The glycerol conversion and the selectivity of the various products have been calculated as follows:

$$\text{Conversion} = 100 \text{ X } [(\text{sum of number of mole of product recovered in the}$$
$$\text{liquid reaction mixture at the end of reaction multiplied by its carbon}$$

$$\text{Selectivity product} = 100 \text{ X } [(\text{number of mole of product recovered in the}$$
$$\text{liquid reaction mixture at the end of reaction multiplied by its carbon}$$
$$\text{number})/(\text{sum of number of mole of products recovered in the liquid reaction}$$
$$\text{mixture at the end of reaction multiplied by their carbon number}) \, ].$$

$$\text{TOF} = (\text{the number of kilogram of converted glycerol})/(\text{the number of}$$
$$\text{kilogram of initial supported platinum})/(\text{the time of reaction}).$$

Examples 7 and 8

The catalyst preparation was as for examples 1 to 6 excepted that the following alumina based oxide has been used as: mixture of gamma- and delta-$Al_2O_3$ (Scca 5/150) was provided by Sasol as powder and used as received and that the weight ratio of Pt and $WO_3$ in the final Pt/$WO_3$/$Al_2O_3$ catalysts were respectively of 5 wt% (Pt) and 10 wt% (WO3).

The test conditions were the following.

The hydrogenation of glycerol has been carried out in a 100 ml hastelloy C autoclave.

2.25 g catalyst, 30 g of a mixture of glycerol (VWR, vegetal origin, min. 99.5% containing 2.15 g/kg of water), 20 g of an aqueous (Milli-Q water) sodium acetate (0.025M)/acetic acid (0.025M) pH buffer have been placed in the autoclave. Subsequently, the autoclave was sealed and purged with nitrogen (N2, Air Product, 99.998%) by pressurizing and depressurizing several times and then with hydrogen (H2, Air Product, 99.9995%) by pressurizing and depressurizing several times, and then heated to the desired temperature of 160 °C. Meanwhile, the hydrogen pressure was increased to 60 bara and mechanical stirring was set at 1000 rpm. The time zero for the reaction was defined as the time at which the stirring was started. During the reaction, the pressure was always maintained at 60 bara. After a reaction time of 15 h, the reaction was stopped by cooling down the autoclave. The pressure was released. The liquid product was recovered and its pH was measured. The liquid product was then further separated by using polypropylene filter (0.8 $\mu$m of porosity)

and then analyzed by gas chromatography (GC) and by ICP.

The GC analysis was as for examples 1 to 6.

The conditions for carrying out examples 7 and 8 and the results are summarized in Table 2 here below.

Table 1

| Example | NaOH/ glycerol (mol/mol) | pH of initial mixture | pH of liquid recovered product | Glycerol conversion (mol%) | Product selectivity (% mol) | | | | | | | | TOF (kg of converted glycerol/kg of Pt/h) |
|---|---|---|---|---|---|---|---|---|---|---|---|---|---|
| | | | | | 1,3-propane-diol | 1,2-propane-diol | 1-propa-nol | 2-propa-nol | methanol | ethylene glycol | Sum ($C_6H_{12}O_3$, $C_6H_{14}O_3$, Un-identified) | sum $CH_4$, $C_2H_6$, $C_3H_8$ | |
| 1 | 0 | 2.82 | 2.49 | 18.2 | 54.2 | 5.8 | 28.1 | 5.6 | 0.2 | n.d. | 3.7 | 2.4 | 11.7 |
| 2 | 0.00015 | 3.91 | 3.17 | 19.9 | 53.6 | 6.7 | 26.9 | 6.1 | 0.2 | n.d. | 4.2 | 2.3 | 12.8 |
| 3 | 0.00071 | 5.12 | 4.16 | 26.3 | 57.7 | 5.5 | 25.3 | 8.5 | 0.1 | n.d. | 1.2 | 1.7 | 17.0 |
| 4 | 0.00084 | 6.53 | 4.83 | 20.6 | 58.5 | 6.2 | 23.5 | 9.3 | 0.1 | n.d. | 0.8 | 1.6 | 13.3 |
| 5 | 0.00130 | 8.02 | 5.37 | 7.3 | 58.0 | 13.3 | 14.7 | 10.5 | 0.4 | 0.9 | 1.4 | 0.8 | 4.7 |
| 6 | 0.00430 | 11.1 | 8.06 | 1.1 | 14.4 | 63.1 | 6.8 | 6.8 | n.d. | 2.9 | 6.0 | n.d. | 0.7 |
| n.d. = not detected | | | | | | | | | | | | | |

EP 3 178 553 B1

Table 2

| Example | buffer/glycerol (mol/mol) | pH of liquid recovered product | Glycerol conversion (mol%) | Product selectivity (% mol) | | | | leaching of W (%) |
|---|---|---|---|---|---|---|---|---|
| | | | | 1,3-propanediol | 1,2-propanediol | 1-propanol | 2-propanol | |
| 7 | 0 | 3.2 | 29.1 | 56.9 | 6.0 | 28.1 | 9.0 | 3.5 |
| 8 | 0.00153 | 4.0 | 25.2 | 59.3 | 7.1 | 24.4 | 9.2 | 0.3 |

**Claims**

1.  A process for manufacturing 1,3-propanediol from glycerol by reacting glycerol with hydrogen in a reactor in the presence of a supported catalyst, said supported catalyst comprising at least one first element selected from iridium, rhodium, palladium, platinum and any mixture thereof, at least one second element selected from chromium, molybdenum, tungsten, and any mixture thereof, and optionally rhenium, said elements and rhenium being supported on alumina, said process comprising introducing at least one ionic base into said reactor.

2.  The process according to claim 1 wherein the molar ratio between the ionic base and the glycerol introduced in the reactor is higher than or equal to 0.00005 and lower than or equal to 0.004.

3.  The process according to claim 2, wherein the molar ratio between the ionic base and the glycerol introduced in the reactor is higher than or equal to 0.00015 and lower than or equal to 0.002.

4.  The process according to any one of claims 1 to 3 wherein the ionic base is added as an alkali metal oxide, an alkaline-earth metal oxide, an alkali metal hydroxide, an alkaline-earth metal hydroxide, ammonium hydroxide, an alkali metal salt of a carboxylic acid, an alkaline-earth metal salt of a carboxylic acid, an ammonium salt of a carboxylic acid or any mixture thereof.

5.  The process according to claim 4 wherein the ionic base is added as sodium hydroxide, sodium acetate, or any mixture thereof.

6.  The process according to claim 5 wherein the ionic base is added as sodium hydroxide.

7.  The process according to claim 5 wherein the ionic base is added as sodium acetate and wherein sodium acetate is added as an aqueous sodium acetate/acetic acid pH buffer.

8.  The process according to any one of claims 1 to 7, wherein the ionic base is added to the reactor as a mixture with glycerol, water and the catalyst, and wherein the pH of said mixture is higher than or equal to 3 and lower than 8.

9.  The process according to any one of claims 1 to 8, comprising withdrawing from the reactor a liquid mixture with a pH higher than or equal to 2.6 and lower than or equal to 7.

10. The process according to any one of claims 1 to 9, wherein the selectivity of 1,3-propanediol is higher than or equal to 5 mol % and the selectivity of 1,2-propanediol with respect to glycerol is lower than or equal to 35 mol %.

11. The process according to claim 10, wherein the first element is platinum and the second element is tungsten.

12. The process according to any one of claims 1 to 11, wherein the reactor contains at least one liquid phase, and wherein the reaction is carried out in at least one of the following conditions:

    Said liquid phase contains water in a content higher than or equal to 3 g of water per kg of liquid phase and lower than or equal to 900 g/kg of liquid phase,
    A temperature higher than or equal to 70 °C and lower than or equal to 300 °C; and
    A hydrogen partial pressure higher than or equal to 1 bar absolute and lower than or equal to 200 bar absolute.

13. The process according to claim 12, said process being carried out in the continuous mode, wherein the reaction is carried out in a trickle-bed reactor at a residence time of the liquid phase higher than or equal to 5 min and lower than or equal to 25 h.

14. A process for making a polyester comprising obtaining 1,3-propanediol according to the process of any one of claims 1 to 13, and further submitting said 1,3-propanediol to a reaction with a carboxylic acid and/or a carboxylic acid ester.

15. A process for making a polyester fiber comprising obtaining a polyester according to the process of claim 14, and further converting said polyester in to a fiber.

EP 3 178 553 B1

**Patentansprüche**

1. Ein Verfahren zur Herstellung von 1,3-Propandiol aus Glycerin durch Umsetzen von Glycerin mit Wasserstoff in einem Reaktor in Anwesenheit eines geträgerten Katalysators, wobei der geträgerte Katalysator mindestens ein erstes Element ausgewählt aus Iridium, Rhodium, Palladium, Platin und einer beliebigen Mischung daraus, mindestens ein zweites Element ausgewählt aus Chrom, Molybdän, Wolfram und einer beliebigen Mischung daraus, und wahlweise Rhenium umfasst, wobei die Elemente und Rhenium auf Aluminiumoxid geträgert sind, wobei das Verfahren das Einführen mindestens einer ionischen Base in den Reaktor umfasst.

2. Das Verfahren gemäß Anspruch 1, wobei das molare Verhältnis zwischen der ionischen Base und dem Glycerin, welche in den Reaktor eingeführt werden, höher als oder gleich 0,00005 und niedriger als oder gleich 0,004 ist.

3. Das Verfahren gemäß Anspruch 2, wobei das molare Verhältnis zwischen der ionischen Base und dem Glycerin, welche in den Reaktor eingeführt werden, höher als oder gleich 0,00015 und niedriger als oder gleich 0,002 ist.

4. Das Verfahren gemäß einem der Ansprüche 1 bis 3, wobei die ionische Base als ein Alkalimetalloxid, ein Erdalkalimetalloxid, ein Alkalimetallhydroxid, ein Erdalkalimetallhydroxid, Ammoniumhydroxid, ein Alkalimetallsalz einer Carbonsäure, ein Erdalkalimetallsalz einer Carbonsäure, ein Ammoniumsalz einer Carbonsäure oder eine beliebige Mischung daraus zugefügt wird.

5. Das Verfahren gemäß Anspruch 4, wobei die ionische Base als Natriumhydroxid, Natriumacetat oder eine beliebigen Mischung daraus zugefügt wird.

6. Das Verfahren gemäß Anspruch 5, wobei die ionische Base als Natriumhydroxid zugefügt wird.

7. Das Verfahren gemäß Anspruch 5, wobei die ionische Base als Natriumacetat zugefügt wird und wobei Natriumacetat als ein wässriger Natriumacetat/Essigsäure pH-Puffer zugefügt wird.

8. Das Verfahren gemäß einem der Ansprüche 1 bis 7, wobei die ionische Base als eine Mischung mit Glycerin, Wasser und dem Katalysator in den Reaktor zugefügt wird und wobei der pH der Mischung höher als oder gleich 3 und niedriger als 8 ist.

9. Das Verfahren gemäß einem der Ansprüche 1 bis 8, umfassend das Entnehmen einer flüssigen Mischung mit einem pH höher als oder gleich 2,6 und niedriger als oder gleich 7 aus dem Reaktor.

10. Das Verfahren gemäß einem der Ansprüche 1 bis 9, wobei die Selektivität von 1,3-Propandiol höher als oder gleich 5 Mol% ist und die Selektivität von 1,2-Propandiol in Bezug auf Glycerin niedriger als oder gleich 35 Mol% ist.

11. Das Verfahren gemäß Anspruch 10, wobei das erste Element Platin ist und das zweite Element Wolfram ist.

12. Das Verfahren gemäß einem der Ansprüche 1 bis 11, wobei der Reaktor mindestens eine flüssige Phase enthält und wobei die Reaktion unter mindestens einer der folgenden Bedingungen durchgeführt wird:

    Die flüssige Phase enthält Wasser in einer Menge von mehr als oder gleich 3 g Wasser pro kg flüssiger Phase und weniger als oder gleich 900 g/kg flüssiger Phase,
    Einer Temperatur höher als oder gleich 70 °C und niedriger als oder gleich 300 °C; und
    Einem Wasserstoffpartialdruck größer als oder gleich 1 bar absolut und niedriger als oder gleich 200 bar absolut.

13. Das Verfahren gemäß Anspruch 12, wobei das Verfahren im kontinuierlichen Modus durchgeführt wird, wobei die Reaktion in einem Rieselbettreaktor bei einer Verweilzeit der flüssigen Phase von mehr als oder gleich 5 min und weniger als oder gleich 25 h durchgeführt wird.

14. Ein Verfahren zur Herstellung eines Polyesters umfassend das Erhalten von 1,3-Propandiol gemäß dem Verfahren gemäß einem der Ansprüche 1 bis 13 und des Weiteren das Umsetzen von 1,3-Propandiol in einer Reaktion mit einer Carbonsäure und/oder einem Carbonsäureester.

15. Ein Verfahren zur Herstellung einer Polyesterfaser umfassend das Erhalten eines Polyesters gemäß dem Verfahren gemäß Anspruch 14 und des Weiteren die Umwandlung des Polyesters in eine Faser.

**Revendications**

1. Procédé de fabrication de 1,3-propanediol à partir de glycérol par réaction de glycérol avec de l'hydrogène dans un réacteur en présence d'un catalyseur sur support, ledit catalyseur sur support comprenant au moins un premier élément choisi parmi l'iridium, le rhodium, le palladium, le platine et un mélange quelconque de ceux-ci, au moins un deuxième élément choisi parmi le chrome, le molybdène, le tungstène, et un mélange quelconque de ceux-ci, et facultativement du rhénium, lesdits éléments et le rhénium étant soutenus sur un support d'alumine, ledit procédé comprenant l'introduction d'au moins une base ionique dans ledit réacteur.

2. Procédé selon la revendication 1, dans lequel le rapport molaire entre la base ionique et le glycérol introduits dans le réacteur est supérieur ou égal à 0,00005 et inférieur ou égal à 0,004.

3. Procédé selon la revendication 2, dans lequel le rapport molaire entre la base ionique et le glycérol introduits dans le réacteur est supérieur ou égal à 0,00015 et inférieur ou égal à 0,002.

4. Procédé selon l'une quelconque des revendications 1 à 3 dans lequel la base ionique est ajoutée sous la forme d'un oxyde de métal alcalin, d'un oxyde de métal alcalino-terreux, d'un hydroxyde de métal alcalin, d'un hydroxyde de métal alcalino-terreux, d'hydroxyde d'ammonium, d'un sel de métal alcalin d'un acide carboxylique, d'un sel de métal alcalino-terreux d'un acide carboxylique, d'un sel d'ammonium d'un acide carboxylique, ou d'un mélange quelconque de ceux-ci.

5. Procédé selon la revendication 4, dans lequel la base ionique est ajoutée sous la forme d'hydroxyde de sodium, d'acétate de sodium, ou d'un mélange quelconque de ceux-ci.

6. Procédé selon la revendication 5, dans lequel la base ionique est ajoutée sous la forme d'hydroxyde de sodium.

7. Procédé selon la revendication 5, dans lequel la base ionique est ajoutée sous la forme d'acétate de sodium et dans lequel l'acétate de sodium est ajouté sous la forme d'un tampon de pH aqueux acétate de sodium/acide acétique.

8. Procédé selon l'une quelconque des revendications 1 à 7, dans lequel la base ionique est ajoutée au réacteur sous la forme d'un mélange avec du glycérol, de l'eau et le catalyseur, et dans lequel le pH dudit mélange est supérieur ou égal à 3 et inférieur à 8.

9. Procédé selon l'une quelconque des revendications 1 à 8, comprenant le retrait à partir du réacteur d'un mélange liquide ayant un pH supérieur ou égal à 2,6 et inférieur ou égal à 7.

10. Procédé selon l'une quelconque des revendications 1 à 9, dans lequel la sélectivité pour le 1,3-propanediol est supérieure ou égale à 5 % en moles et la sélectivité pour le 1,2-propanediol par rapport au glycérol est inférieure ou égal à 35 % en moles.

11. Procédé selon la revendication 10, dans lequel le premier élément est le platine et le deuxième élément est le tungstène.

12. Procédé selon l'une quelconque des revendications 1 à 11, dans lequel le réacteur contient au moins une phase liquide, et dans lequel la réaction est conduite dans au moins l'une des conditions suivantes :

   ladite phase liquide contient de l'eau à une teneur supérieure ou égale à 3 g d'eau par kg de phase liquide et inférieure ou égale à 900 g/kg de phase liquide,
   une température supérieure ou égale à 70 °C et inférieure ou égale à 300 °C ; et
   une pression partielle d'hydrogène supérieure ou égale à 1 bar absolu et inférieure ou égal à 200 bars absolus.

13. Procédé selon la revendication 12, ledit procédé étant conduit dans le mode continu, dans lequel la réaction est conduite dans un réacteur à lit ruisselant avec un temps de séjour de la phase liquide supérieur ou égal à 5 min et inférieur ou égal à 25 h.

14. Procédé de fabrication d'un polyester comprenant l'obtention de 1,3-propanediol selon le procédé selon l'une quelconque des revendications 1 à 13, et en outre, la soumission dudit 1,3-propanediol à une réaction avec un acide carboxylique et/ou un ester d'acide carboxylique.

15. Procédé de fabrication d'une fibre de polyester comprenant l'obtention d'un polyester selon le procédé de la revendication 14 et en outre, la conversion dudit polyester en une fibre.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Effect of Base Additives on the Selective Hydrogenolysis of Glycerol over Ru/TiO2 Catalyst. **FENG JIAN et al.** Chemistry Let. Chemical society of Japan, vol. 36, 1274-1275
- **L.K. HUDSON et al.** Ullmann's Encyclopedia of Industrial Chemistry. 15 June 2000, 607-645
- **NAGAWA et al.** *Journal of Catalysis,* 2010, vol. 272, 191-194
- Ullmann's Encyclopedia of Industrial Chemistry. Wiley-VCH Verlag GmbH & Co, 2005, 8-15
- **HORST KÖPNICK ; MANFRED SCHMIDT ; WILHELM BRÜGGING ; JÖRN RÜTER ; WALTER KAMINSKY.** Ullmann's Encyclopedia of Industrial Chemistry. 15 June 2000, 623-649
- **HELMUT SATTLER ; MICHAEL SCHWEIZER.** Ullmann's Encyclopedia of Industrial Chemistry. 15 October 2011, 1-34